# EUROPEAN PATENT SPECIFICATION

(11) **EP 3 484 996 B1**
(45) Date of publication and mention of the grant of the patent: **09.09.2020**
(21) Application number: 17736682.0
(22) Date of filing: 12.07.2017
(51) Int. Cl.: C12N 1/20, C12N 1/38, C12N 1/00, C12N 1/16, C12N 1/18, C12N 1/14

(54) **FERMENTATION MEDIUM COMPRISING CHELATING AGENT**
FERMENTATIONSMEDIUM MIT CHELATBILDNER
MILIEU DE FERMENTATION COMPRENANT UN AGENT CHÉLATEUR

(30) Priority: 14.07.2016 EP 16179547
(43) Date of publication of application: 22.05.2019
(73) Proprietor: BASF SE, 67056 Ludwigshafen am Rhein (DE)
(72) Inventor: HAGE, Kerstin, 31582 Nienburg (DE); SCHWALB, Carsten, 67056 Ludwigshafen (DE); STEIGELMANN, Gunter, 67056 Ludwigshafen (DE); KAEDING, Thomas, 67056 Ludwigshafen (DE); BRIECHLE, Sebastian, 67056 Ludwigshafen (DE)
(74) Representative: BASF IP Association
(86) International application number: PCT/EP2017/067474
(87) International publication number: WO 2018/011242

(56) References cited:
- EP-A1- 2 660 315
- EP-A1- 2 944 696
- WO-A1-92/14848
- WO-A1-2009/041831
- WO-A1-2013/000844
- WO-A1-2015/036375
- WO-A1-2016/034691
- WO-A2-2005/005773
- WO-A2-2015/111972
- CN-A- 105 271 514
- DE-A1- 2 925 427
- KR-A- 20130 134 846
- US-A1- 2013 012 383
- US-A1- 2013 224 839
- US-B1- 6 329 192
- HASEGAWA H. ET AL: "Influence of chelating ligands on bioavailability and mobility of iron in plant growth media and their effect on radish growth", ENVIRONMENTAL AND EXPERIMENTAL BOTANY, vol. 71, no. 3, 1 July 2011 (2011-07-01), pages 345-351, XP028166502, ISSN: 0098-8472, DOI: 10.1016/J.ENVEXPBOT.2011.01.004
- AZIZUR RAHMAN M. ET AL: "The significance of biodegradable methylglycinediacetic acid (MGDA) for iron and arsenic bioavailability and uptake in rice plant", SOIL SCIENCE AND PLANT NUTRITION, vol. 58, no. 5, 1 October 2012 (2012-10-01), pages 627-636, XP055400214, JP ISSN: 0038-0768, DOI: 10.1080/00380768.2012.717246
- BRÄUER S. L. ET AL: "Characterization of acid-tolerant H2/CO2-utilizing methanogenic enrichment cultures from an acidic peat bog in New York State", FEMS MICROBIOLOGY ECOLOGY, vol. 57, no. 2, 8 June 2006 (2006-06-08), pages 206-216, XP055330974, NL ISSN: 0168-6496, DOI: 10.1111/j.1574-6941.2006.00107.x
- VAN GINKEL C. G. ET AL: "Biodegradation pathway of L-glutamatediacetate by Rhizobium radiobacter strain BG-1", INTERNATIONAL BIODETERIORATION & BIODEGRADATION, vol. 62, no. 1, 26 December 2007 (2007-12-26), pages 31-37, XP022707468, ISSN: 0964-8305, DOI: 10.1016/J.IBIOD.2007.08.003 [retrieved on 2007-12-26]
- KAKII K. ET AL: "Isolation and growth characteristics of nitrilotriacetate-degrading bacteria", JOURNAL OF FERMENTATION TECHNOLOGY, vol. 64, no. 2, 1986, pages 103-108, XP023543011, ISSN: 0385-6380, DOI: 10.1016/0385-6380(86)90004-X [retrieved on 1986-01-01]
- NARSIAN V. ET AL: "Aspergillus aculeatus as a rock phosphate solubilizer", SOIL BIOLOGY AND BIOCHEMISTRY, vol. 32, no. 4, 1 April 2000 (2000-04-01), pages 559-565, XP055330978, GB ISSN: 0038-0717, DOI: 10.1016/S0038-0717(99)00184-4
- JEGO P. ET AL: "Inhibition of iron overload toxicity in rat hepatocyte cultures by pyoverdin Pf, the siderophore of pseudomonas fluorescens", BIOCHEMICAL PHARMACOLOGY, vol. 43, no. 6, 17 March 1992 (1992-03-17) , pages 1275-1280, XP025542472, ISSN: 0006-2952, DOI: 10.1016/0006-2952(92)90503-B [retrieved on 1992-03-17]

## Description

### Field of the invention

The present invention is directed to a fermentation medium and a fermentation method using said fermentation medium wherein the fermentation medium comprises a basal medium and a chelating agent having the formula (I) R¹-CH(COOX¹)-N(CH₂COOX¹)₂, wherein R¹ is methyl, X¹ is (MₓH₁₋ₓ), M being selected from ammonium or alkali metal or combinations of at least two of the foregoing, x is in the range of from zero to 1, wherein x is an average value.

### Background of the invention

The biotechnological production of useful substances by cultivation of selected microorganisms producing these substances is now of considerable industrial significance. Especially, the industrial production of proteins, in particular washing- and/or cleaning-active enzymes, but also pharmacologically active compounds by fermentation has increased over the past decades.

For industrial scale fermentation, the cultured microorganism usually requires a complex mixtures of nutrients. Among others, the production strain usually requires during fermentation a nitrogen and a carbon source, salts comprising for instance a source of phosphor and sulfur as well as trace elements. In this complex mixture and under the pH conditions used precipitation of insoluble components, e.g., salts, usually occurs during preparation of the fermentation media as well as during cultivation. This precipitation is disadvantageous as it leads to a loss of accessibility of the nutrients comprised in these precipitates. As majority of these precipitates do not dissolve they represent a loss of assets. Moreover, the formation of precipitates causes inhomogeneity and makes stirring of the fermentation broth more difficult resulting in an increased energy consumption of the fermentation process. In addition, precipitates disturb purification of compounds of interest from the fermentation broth because the precipitates interfere with subsequent downstream processing operations.

This problem is solved by the addition of a chelating agent to the fermentation medium, which is capable of binding certain ions, mainly cations, and thus, reduces the formation of insoluble precipitates. Usually citrate is used as a low cost chelating agent in the fermentation medium. Although citrate is reasonably efficient in ion complexation still a significant amount of precipitation of insolubles is frequently observed when citrate is used in fermentation media with high salt loads. In addition, most microorganism are capable of metabolizing citrate during the fermentation process resulting in a significant breakdown of the chelating agent by the microorganism. In turn, chelation efficiency decreases during the course of fermentation and additional precipitation occurs.

An alternative well-known chelating agent is EDTA. However, EDTA and its derivatives are associated with the disadvantage of a high binding affinity to heavy metal ions, like arsenic, cadmium, and mercury. This in turn leads to an increased leakage of such heavy metal ions during the course of decomposition of fermentation waste in waste water treatment plants.

Thus, there was the need for a fermentation medium comprising a chelating agent with improved complexation efficiency and with a reduced tendency to being metabolized by the cultivated microorganism.

The solution to the described problem is provided by the present invention, which is directed to a fermentation medium and a fermentation method using said fermentation medium wherein the fermentation medium comprising a basal medium and a chelating agent having the formula R¹-CH(COOX¹)-N(CH₂COOX¹)₂ (I), wherein R¹ is methyl, X¹ is (MₓH₁₋ₓ), M being a monovalent cation, preferably being selected from ammonium or alkali metal or combinations of at least two of the foregoing, x is in the range of from zero to 1, wherein x is an average value.

Chelating agents of the aminocarboxylate type according to the above mentioned formula (I) and their respective salts are useful sequestrants for preferably alkaline earth metal ions such as Ca²⁺ and Mg²⁺. Compounds of this type show good biodegradability and are thus environmentally friendly. Moreover, these compounds show low toxicity to the cultivated microorganism in the concentration range used during fermentation. For these reasons, they are recommended and used for various purposes such as laundry detergents and for automatic dishwashing (ADW) formulations, in particular for so-called phosphate-free laundry detergents and phosphate-free ADW formulations (cf., e.g., WO2006002954A1 and WO2013160259A1). Compounds according to the above mentioned formula (I) have also been disclosed to be used for improving the efficiency of herbicides (cf. WO2014206835A1). A method of producing such components is described for instance in WO2015089012A1.

However, none of the cited prior art teaches or suggests the use of a compound according to formula (I) as a chelating agent in a fermentation medium.

WO 92/14848 A1, EP 2944696 A1, WO 2016/034691 A1, US 2013/224839 A1, US 6329192 B1, CN 105271514 A, BRÄUER S.L. et al. (2006) FEMS MICROBIOL ECOL 57(2):206-216, WO 2009/041831 A1, WO 2005/005773 A2, VAN GINKEL C.G. et al. (2007) INT BIODETERIOR BIODEGRAD 62(1):31-37, WO 2015/111972 A2, and KAKII K. et al. (1986) J FERMENT TECHNOL 64(2):103-108 disclose bacterial fermentation media containing nitrilotriacetic acid (NTA) as chelating agent. Of these, WO 2016/034691 A1, US 2013/224839 A1, CN 105271514 A, BRÄUER S.L. et al. (2006) FEMS MICROBIOL ECOL 57(2):206-216, WO 2009/041831 A1, and WO 2005/005773 A2 also disclose trace element solutions containing NTA as chelating agent. VAN GINKEL C.G. et al. (2007) INT BIODETERIOR BIODEGRAD 62(1):31-37 also discloses bacterial fermentation media containing glutamatediacetate (GLDA) as chelating agent.

### Brief summary of the invention

The present invention is directed to a fermentation medium and a fermentation method using said fermentation medium wherein the fermentation medium comprising a basal medium and a chelating agent having the formula R¹-CH(COOX¹)-N(CH₂COOX¹)₂ (I), wherein R¹ is methyl, X¹ is (MₓH₁₋ₓ), M being a monovalent cation, preferably being selected from ammonium, alkali metal and combinations of at least two of the foregoing, x is in the range of from zero to 1, wherein x is an average value.

In particular, the present invention is directed to a method of cultivating a microorganism using a fermentation medium comprising a basal medium and a chelating agent having the formula R¹-CH(COOX¹)-N(CH₂COOX¹)₂ (I), wherein R¹ is methyl, X¹ is (MₓH₁₋ₓ), M being a monovalent cation, preferably being selected from ammonium, alkali metal and combinations of at least two of the foregoing, x is in the range of from zero to 1, wherein x is an average value.

Furthermore, the present invention is directed to a method of producing a compound of interest by cultivating a microorganism using a fermentation medium comprising a basal medium and a chelating agent having the formula R¹-CH(COOX¹)-N(CH₂COOX¹)₂ (I), wherein R¹ is methyl, X¹ is (MₓH₁₋ₓ), M being a monovalent cation, preferably being selected from ammonium, alkali metal and combinations of at least two of the foregoing, x is in the range of from zero to 1, wherein x is an average value.

Moreover, the present invention is directed to the use of a chelating agent having the formula R¹-CH(COOX¹)-N(CH₂COOX¹)₂ (I), wherein R¹ is methyl, X¹ is (MₓH₁₋ₓ), M being a monovalent cation, preferably being selected from ammonium, alkali metal and combinations of at least two of the foregoing, x is in the range of from zero to 1, wherein x is an average value, for the preparation of a fermentation medium, for the cultivating of a microorganism, and for the production of a compound of interest.

### Brief description of the Figures

Figure 1: Scatter plot curves as measure for biomass growth for cultivations in µL scale for media with different chelating agents.
Figure 2: Comparison of enzyme production in AU [-] with different chelating agents (citric acid, MGDA, NTA, HEEDTA, or EGTA) in the trace element solution with pH 6.7 after 50 h of cultivation in µL scale
Figure 3: Comparison of enzyme production in AU [-] with different chelating agents (citric acid, MGDA, NTA, HEEDTA, or EGTA) in the trace element solution with pH 7.7 after 50 h of cultivation in µL scale.

### Detailed description of the invention

The present invention may be understood more readily by reference to the following detailed description of the preferred embodiments of the invention and the examples included herein.

### Definitions

Unless otherwise noted, the terms used herein are to be understood according to conventional usage by those of ordinary skill in the relevant art.

It is to be understood that as used in the specification and in the claims, "a" or "an" can mean one or more, depending upon the context in which it is used. Thus, for example, reference to "a cell" can mean that at least one cell can be utilized.

The term "basal medium" is used herein for any type of liquid fermentation medium suitable for growth of a cell (e.g., a cell of a microorganism, e.g., a bacterial cell and/or a fungal cell).

The term "complex nitrogen source" is used herein for a component of a fermentation medium that comprises different nitrogen containing compounds, preferably comprising organic nitrogen containing compounds, e.g., different proteins and/or amino acids, and which can comprise up to 49% of carbohydrates, preferably 0.5-20% carbohydrates.

The term "complex carbon source" is used herein for a component of a fermentation medium that comprises different carbon containing compounds, e.g., carbohydrates, and which can comprise up to 49% of nitrogen comprising compounds, preferably 0.5-20% nitrogen comprising compounds.

The term "fermentation in industrial scale" (also called large-scale fermentation) refers to fermentation processes with fermenter volumes of greater than or equal to 20 liters.

The term "equimolar" refers to a relative concentration of the chelating agent with respect to the multivalent cations, preferably divalent and trivalent, more preferably divalent, present in the solution, wherein the molar concentration of the chelating agent is the same as the molar concentration of the sum of the divalent salt cations in the solution, wherein salt cations contained in complex nitrogen or carbon sources are not included.

### Detailed description

The present invention is directed to a fermentation medium comprising a basal medium and a chelating agent having the formula (I)

R¹-CH(COOX¹)-N(CH₂COOX¹)₂

wherein
- R1: is methyl,
- X¹: is (MₓH₁₋ₓ), M being a monovalent cation, preferably being selected from ammonium, alkali metal and combinations of at least two of the foregoing,

Also disclosed herein is a fermentation medium comprising a basal medium and a chelating agent having the formula (I)

R¹-CH(COOX¹)-N(CH₂COOX¹)₂

wherein
- R1: is selected from
hydrogen,
linear or branched C₁-C₄-alkyl,
phenyl, benzyl, CH2OH, and CH2CH2COOX1,
- X¹: is (MₓH₁₋ₓ), M being a monovalent cation, preferably being selected from ammonium, alkali metal and combinations of at least two of the foregoing,
- x: is in the range of from zero to 1, wherein x is an average value.

Preferably, R¹ is selected from hydrogen, C₁-C₄-alkyl, linear or branched, for example methyl, ethyl, n-propyl, iso-propyl, n-butyl, iso-butyl, sec.-butyl, further preferred are methyl and iso-butyl and sec.-butyl and even more preferred is methyl. Also, preferred is R¹ being phenyl, benzyl, CH₂OH, or CH₂CH₂COOX¹, preferred is CH₂CH₂COOX¹. In particular preferred are compounds according to formula (I) with R¹ of formula (I) selected from hydrogen, linear or branched C₁-C₄-alkyl, and CH₂CH₂COOX¹. Further preferred are compounds with R¹ of formula (I) being selected from hydrogen and linear or branched C₁-C₄-alkyl, preferably, methyl. Even more preferred are compounds with R¹ of formula (I) being selected from the group consisting of hydrogen, methyl and CH₂CH₂COOX¹. Preferably, R¹ of formula (I) is hydrogen. In a preferred embodiment, R¹ is not a carboxyl group. Further preferred is R¹ of formula (I) being methyl. Also preferred is R¹ of formula (I) being CH₂CH₂COOX¹. Also mixtures of different chelating agents with at least one falling under formula (I) can be added to the fermentation medium. Preferably, at least two different chelating agents falling under formula (I) with different R¹ identities can be added to the fermentation medium.

The fermentation medium disclosed herein preferably comprises a basal medium and a chelating agent having the formula (I)

R¹-CH(COOX¹)-N(CH₂COOX¹)₂

wherein
- R¹: is selected from
linear or branched C₁-C₄-alkyl,
phenyl, benzyl, and CH₂OH,
- X¹: is (MₓH₁₋ₓ), M being a monovalent cation, preferably being selected from ammonium or alkali metal or combinations of at least two of the foregoing and x being in the range of from zero to 1, wherein x is an average value.

Also disclosed herein is a fermentation medium comprising a basal medium and a chelating agent having the formula (I)

R¹-CH(COOX¹)-N(CH₂COOX¹)₂

wherein
- R¹: is selected from
linear or branched C₁-C₄-alkyl,
- X¹: is (MₓH₁₋ₓ), M being a monovalent cation, preferably being selected from ammonium or alkali metal or combinations of at least two of the foregoing and x being in the range of from zero to 1, wherein x is an average value.

According to the present invention, the fermentation medium comprises a basal medium and a chelating agent having the formula (I)

R¹-CH(COOX¹)-N(CH₂COOX¹)₂

wherein
- R¹: is methyl,
- X¹: is (MₓH₁₋ₓ), M being a monovalent cation, preferably being selected from ammonium or alkali metal or combinations of at least two of the foregoing and x being in the range of from zero to 1, wherein x is an average value.

In a preferred embodiment, M is selected from ammonium, alkali metal and combinations of at least two of the foregoing. In a more preferred embodiment, M is selected from alkali metal, for example lithium, sodium or potassium, rubidium, cesium, or combinations of at least two of the foregoing, preferred is potassium or sodium and combinations of sodium and potassium, and sodium is even more preferred.

In a preferred embodiment, x is in the range of from zero to 1, wherein x is an average value. Preferably, x is from 0.6 to 1, more preferably x is zero or 1, preferably x is 1.

In a preferred embodiment, R¹ is methyl, x is 1, and X¹ is selected from potassium and sodium and combinations of sodium and potassium, and preference being given to sodium.

Preferably, examples of chelating agents used in the present invention are compounds according to general formula (II)

[R¹-CH(COO)-N(CH₂-COO)₂]M₃₋ₓHₓ (II)

wherein
M is selected from ammonium, alkali metal cations, same or different, for example cations of lithium, sodium, potassium, rubidium, cesium, and combinations of at least two of the foregoing, preferred alkali metal cations are sodium and potassium, preferably sodium, and combinations of sodium and potassium;
x is in the range of from zero to 3.0, wherein x is an average value, preferably in the range of from zero to 2.0, more preferred in the range of zero to 1.0, preferred are zero to 0.5, in a particularly preferred embodiment, x is 1 or zero;
R¹ is methyl.

Further disclosed examples of chelating agents are compounds according to general formula (II)

[R¹-CH(COO)-N(CH₂-COO)₂]M₃₋ₓHₓ (II)

wherein
M is selected from ammonium, alkali metal cations, same or different, for example cations of lithium, sodium, potassium, rubidium, cesium, and combinations of at least two of the foregoing, preferred alkali metal cations are sodium and potassium, preferably sodium, and combinations of sodium and potassium;
x is in the range of from zero to 3.0, wherein x is an average value, preferably in the range of from zero to 2.0, more preferred in the range of zero to 1.0, preferred are zero to 0.5, in a particularly preferred embodiment, x is 1 or zero;
R¹ is selected from hydrogen, C₁-C₄-alkyl, for example methyl, ethyl, iso-propyl, sec.-butyl and iso-butyl, preferably hydrogen or methyl.

In another embodiment preferred examples of chelating agents are compounds according to general formula (III)

[OOC-CH₂CH₂C-CH(COO)-N(CH₂-COO)₂]M₄₋ₓHₓ (III)

wherein
M is selected from ammonium, alkali metal cations, same or different, for example cations of lithium, sodium, potassium, rubidium, cesium, and combinations of at least two of the foregoing, preferred alkali metal cations are sodium and potassium, preferably sodium, and combinations of sodium and potassium,
x is in the range of from zero to 4.0, wherein x is an average value, preferably in the range of from zero to 3.0, more preferred in the range of zero to 2.0, further preferred from zero to 1.0, preferred are zero to 0.5, in a particularly preferred embodiment, x is 1 or zero.

In a preferred embodiment, R¹ in formula (I) is methyl. Compounds of general formula (I) wherein R¹ is methyl are hereinafter also referred to as MGDA, methyl glycine diacetic acid.

In a preferred embodiment, the chelating agent is methyl glycine diacetic acid (MGDA).

Within the disclosure of this application, the chelating agent is selected from the group consisting of nitrilotriacetic acid (NTA), methyl glycine diacetic acid (MGDA), glutamic acid N,N-diacetic acid (GLDA) and salts thereof, and combinations thereof.

In another preferred embodiment, the fermentation medium of the present invention comprising a basal medium and a chelating agent according to formula (I) as described herein, wherein the fermentation medium does not comprise citric acid and/or a salt thereof. Preferably, the fermentation medium does not comprise one or more of the chelating agents selected from the group consisting of ethylene diamine tetraacetic acid (EDTA), diethylene triamine pentaacetic acid (DTPA), hydroxyethylethylene diamine triacetic acid (HEEDTA), and ethylene glycol tetraacetic acid (EGTA). Preferably, fermentation medium does not comprise one or more of the chelating agents selected from the group consisting of citrate, EDTA, DTPA, HEEDTA, and EGTA. More preferred, the fermentation medium does not comprise citrate and EDTA. Preferably, no citrate except citrate contained in one or more of the complex nitrogen or carbon sources is comprised in the fermentation medium.

In a preferred embodiment, the chelating agent of the fermentation medium described herein is readily biodegradable (based on OECD criteria, e.g., determined according to OECD guideline 301F, 301B, ISO9439, 92/69/EEC, C,4-C), preferably 80-100%, more preferably 80-90% or 90-100% BOD of the ThOD (28d).

In a preferred embodiment, the chelating agent of the fermentation medium described herein is not a natural substrate for the cultivated microorganism, preferably, the cultivated microorganism is not able to metabolize the chelating agent.

In one embodiment of the present invention, compound of general formula (I), (II), or (III) is the racemic mixture. In other embodiments, compound of general formula (I), (II), or (III) is selected from the pure enantiomers, for example the L-enantiomer, or mixtures of enantiomers in which one of the enantiomers prevails, preferably the L-enantiomer.

In one embodiment of the present invention, compound of general formula (I), (II), or (III) is selected from mixtures of enantiomers containing predominantly the respective L-isomer with an enantiomeric excess (ee) in the range of from 3 to 97 %, preferably 20 to 80% and even more preferably 25 to 75%. Preferably, the molar ratio of L/D enantiomer is in the range of from 55:45 to 99:1, more preferably from 80:20 to 99:1.

In one embodiment of the present invention, alkali metal salts of MGDA may be selected from alkali metal salts of the L-enantiomer, of the racemic mixture and of enantiomerically enriched alkali metal salts of MGDA, with an excess of L-enantiomer compared to the D-enantiomer. Preference is given to alkali metal salts of mixtures from the L-enantiomer and the D-enantiomer in which the molar ratio of L/D is in the range of from 55:45 to 85:15. Such mixtures exhibit a lower hygroscopicity than, e.g., the racemic mixture.

Alkali metal salts of GLDA may be selected from alkali metal salts of the L-enantiomer, of the racemic mixture and of enantiomerically enriched GLDA, with an excess of L-enantiomer compared to the D-enantiomer. Preference is given to alkali metal salts of mixtures from L-enantiomer and D-enantiomer in which the molar ratio of L/D is in the range of from 80:20 or higher, preferably of from 85:15 up to 99:1. Such alkali metal salts of GLDA have a better biodegradability than, e.g., the racemic mixture or the pure D-enantiomer.

The enantiomeric excess can be determined by measuring the polarization (polarimetry) or preferably by chromatography, for example by HPLC with a chiral column, for example with one or more cyclodextrins as immobilized phase or with a ligand exchange (Pirkle-brush) concept chiral stationary phase. Preferred is determination of the ee by HPLC with an immobilized optically active amine such as D-penicillamine in the presence of copper(II) salt, especially for compounds according to general formula (I) or (II) with R¹ being methyl.
In the context of the present invention, alkali metal salts of compounds of formula (I), (II), or (III) are selected from lithium salts, potassium salts and preferably sodium salts. A compound of formula (I), (II), or (III) can be partially or preferably fully neutralized with the respective alkali. In a preferred embodiment, an average of from 2.7 to 3 COOH groups of a compound of formula (I), (II), or (III) is neutralized with alkali metal, preferably with sodium. In a particularly preferred embodiment, chelating agent (A) is the trisodium salt of a compound of formula (I), (II), or (III). Preferably, the chelating agent according to formula (I), (II), or (III) as described herein is used in the fermentation medium in a concentration equimolar with respect to the multivalent, preferably, divalent cations and trivalent cations, preferably divalent cations, contained in the fermentation medium. Preferably, the concentration is at least 50% equimolar, more preferably at least 60%, at least 70%, at least 75%, at least 80%, at least 85%, at least 90%, at least 95%, at least 100%, at least 110%, at least 120%, at least 130%, at least 140%, at least 150%, at least
175%, or at least 200% equimolar. Preferably the chelating agent is used in a concentration of 0.1 - 3.0 g/L, more preferably 0.2 - 2 g/L, most preferably 0.2 - 1.5 g/L, even more preferred 0.5 - 1.5 g/L.

Culturing a microorganism frequently requires that cells be cultured in a medium containing various nutrition sources, like carbon source, nitrogen source, and other nutrients e.g., amino acids, vitamins, minerals, etc., required for growth of those cells. The fermentation medium may be a minimal medium as described in, e.g., WO 98/37179, or the fermentation medium may be a complex medium comprising complex nitrogen and carbon sources, wherein the complex nitrogen source may be partially hydrolyzed as described in WO 2004/003216.

Thus, fermentation medium of the present invention comprises a basal medium, wherein preferably the basal medium comprises components required for the growth of the cultivated microorganism. Preferably, the basal medium comprises one or more components selected from the group consisting of nitrogen source, phosphor source, sulfur source and salt, and optionally one or more further components selected the group consisting of micronutrients, like vitamins, amino acids, minerals, and trace elements. Preferably, the basal medium also comprises a carbon source. Such components are generally well known in the art (see, e.g., Ausubel, et al, Short Protocols in Molecular Biology, 3rd ed., Wiley & Sons, 1995; Sambrook, et al., Molecular Cloning: A Laboratory Manual, Second Edition, 1989 Cold Spring Harbor, N.Y.; Talbot, Molecular and Cellular Biology of Filamentous Fungi: A Practical Approach, Oxford University Press, 2001; Kinghom and Turner, Applied Molecular Genetics of Filamentous Fungi, Cambridge University Press, 1992; and Bacillus (Biotechnology Handbooks) by Colin R. Harwood, Plenum Press, 1989). Culture conditions for a given cell type may also be found in the scientific literature and/or from the source of the cell such as the American Type Culture Collection (ATCC) and Fungal Genetics Stock Center.

As sources of nitrogen, inorganic and organic nitrogen compounds may be used, both individually and in combination. Illustrative examples of suitable nitrogen sources include protein-containing substances, such as an extract from microbial, animal or plant cells, e.g., plant protein preparations, soy meal, corn meal, pea meal, corn gluten, cotton meal, peanut meal, potato meal, meat and casein, gelatins, whey, fish meal, yeast protein, yeast extract, tryptone, peptone, bacto-tryptone, bacto-peptone, wastes from the processing of microbial cells, plants, meat or animal bodies, and combinations thereof. Examples of inorganic nitrogen sources are ammonium, nitrate, and nitrit, and combinations thereof. In a preferred embodiment, the fermentation medium comprises a nitrogen source, wherein the nitrogen source is a complex or a defined nitrogen source or a combination thereof. Preferably, the complex nitrogen source is selected from the group consisting of plant protein, preferably potato protein, soy protein, corn protein, peanut, cotton protein, and/or pea protein, casein, tryptone, peptone and yeast extract and combinations thereof. Preferably, the defined nitrogen source is selected from the group consisting of ammonia, ammonium, ammonium salts, (e.g., ammonium chloride, ammonium nitrate, ammonium phosphate, ammonium sulfate, ammonium acetate), urea, nitrate, nitrate salts, nitrit, and amino acids, preferably, glutamate, and combinations thereof.

In a preferred embodiment, the fermentation medium further comprises a carbon source. The carbon source is preferably a complex or a defined carbon source or a combination thereof. Various sugars and sugar-containing substances are suitable sources of carbon, and the sugars may be present in different stages of polymerisation. Preferred complex carbon sources to be used in the present invention are selected from the group consisting of molasse, corn steep liquor, cane sugar, dextrin, starch, starch hydrolysate, and cellulose hydrolysate, and combinations thereof. Preferred defined carbon sources are selected from the group consisting of carbohydrates, organic acids, and alcohols, preferably, glucose, fructose, galactose, xylose, arabinose, sucrose, maltose, lactose, acetic acid, propionic acid, lactic acid, formic acid, malic acid, citric acid, fumaric acid, glycerol, inositol, mannitol and sorbitol, and combinations thereof. Preferably, the defined carbon source is provided in form of a sirup, which can comprise up to 20%, preferably, up to 10%, more preferably up to 5% impurities. Preferably, the carbon source is sugar beet sirup, sugar cane sirup, corn sirup, preferably, high fructose corn sirup. Preferably, the complex carbon source is selected from the group consisting of molasses, corn steep liquor, dextrin, and starch, or combinations thereof, and wherein the defined carbon source is selected from the group consisting of glucose, fructose, galactose, xylose, arabinose, sucrose, maltose, dextrin, lactose, or combinations thereof.

Preferably, the basal medium also comprises a phosphor source, preferably, phosphate salts, and preferably, a sulfur source, preferably, sulfate salts. Preferably, the basal medium also comprises a salt, preferably, an inorganic salts, in particular one or more salts selected from the group consisting of alkali metal salts, alkali earth metal salts, phosphate salts and sulfate salts, preferably selected from the group consisting of NaCl, KH2PO4, MgSO4, CaCI2, FeCl3, MgCI2, MnCI2, ZnSO4, Na2MoO4 and CuSO4. Preferably, the basal medium also comprises one or more vitamins, e.g., thiamine chloride, biotin, vitamin B12. Preferably, the basal medium also comprises trace elements, such as Fe, Mg, Mn, Co, and Ni. Preferably, the fermentation medium comprises one or more salt cations selected from the group consisting of Na, K, Ca, Mg, Mn, Fe, Co, Cu, and Ni, preferably, divalent or trivalent cations, more preferably, Ca and Mg. Preferably, the fermentation medium comprises at least two, at least three, at least four, at least five, at least six, at least seven, at least eight, or all of the salt cations selected from the group consisting of Na, K, Ca, Mg, Mn, Fe, Co, Cu, and Ni. Preferably, the fermentation medium comprises the salt cations Na, Ca, and Mg, more preferably Na, Ca, Mg, and Mn, even more preferably Na, K, Ca, Mg, Mn, and Co, Cu, and/or Ni.

In particular, the present invention is directed to a fermentation medium comprising a basal medium and a chelating agent having the formula R¹-CH(COOX¹)-N(CH₂COOX¹)₂ (I), wherein R¹ is methyl, X¹ is (MₓH₁₋ₓ), M being a monovalent cation, preferably being selected from ammonium or alkali metal or combinations of at least two of the foregoing, x is in the range of from zero to 1, wherein x is an average value and wherein the a fermentation medium comprises one or more salt cations, preferably divalent or trivalent salt cations, preferably trace elements, more preferably one or more salt cations selected from the group consisting of Na, K, Ca, Mg, Mn, Fe, Co, Cu, and Ni, most preferably, Ca and Mg.

Disclosed herein is a fermentation medium comprising a basal medium and a chelating agent having the formula R¹-CH(COOX¹)-N(CH₂COOX¹)₂ (I), wherein R¹ is selected from hydrogen, linear or branched C₁-C₄-alkyl, phenyl, benzyl, CH₂OH, and CH₂CH₂COOX¹, X¹ is (MₓH₁₋ₓ), M being a monovalent cation, preferably being selected from ammonium or alkali metal or combinations of at least two of the foregoing, x is in the range of from zero to 1, wherein x is an average value and wherein the a fermentation medium comprises one or more salt cations, preferably divalent or trivalent salt cations, preferably trace elements, more preferably one or more salt cations selected from the group consisting of Na, K, Ca, Mg, Mn, Fe, Co, Cu, and Ni, most preferably, Ca and Mg.

Preferably, the chelating agent is dissolved directly in the fermentation medium. In another preferred embodiment a solution comprising the chelating agent is prepared, e.g., a stock solution, which is subsequently added to the fermentation medium. Preferably, the chelating agent is dissolved in a salt solution, preferably, a trace element solution, which is subsequently added to the fermentation medium.

Disclosed herein is a trace element solution comprising one or more trace elements and a chelating agent having the formula (I)

R¹-CH(COOX¹)-N(CH₂COOX¹)₂

wherein
- R¹: is selected from
hydrogen,
linear or branched C₁-C₄-alkyl,
phenyl, benzyl, CH₂OH, and CH₂CH₂COOX¹,
- X¹: is (MₓH₁₋ₓ), M being a monovalent cation, preferably being selected from ammonium or alkali metal or combinations of at least two of the foregoing and x being in the range of from zero to 1, wherein x is an average value.

Preferably, the trace element solution comprises one or more trace elements and a chelating agent having the formula (I)

R¹-CH(COOX¹)-N(CH₂COOX¹)₂

wherein
- R¹: is selected from
linear or branched C₁-C₄-alkyl,
phenyl, benzyl, and CH₂OH,
- X¹: is (MₓH₁₋ₓ), M being a monovalent cation, preferably being selected from ammonium or alkali metal or combinations of at least two of the foregoing and x being in the range of from zero to 1, wherein x is an average value.

In a further embodiment, the trace element solution comprises one or more trace elements and a chelating agent having the formula (I)

R¹-CH(COOX¹)-N(CH₂COOX¹)₂

wherein
- R¹: is selected from
linear or branched C₁-C₄-alkyl,
- X¹: is (MₓH₁₋ₓ), M being a monovalent cation, preferably being selected from ammonium or alkali metal or combinations of at least two of the foregoing and x being in the range of from zero to 1, wherein x is an average value.

In a further embodiment, the trace element solution comprises one or more trace elements and a chelating agent having the formula (I)

R¹-CH(COOX¹)-N(CH₂COOX¹)₂

wherein
- R¹: is methyl,
- X¹: is (MₓH₁₋ₓ), M being a monovalent cation, preferably being selected from ammonium or alkali metal or combinations of at least two of the foregoing and x being in the range of from zero to 1, wherein x is an average value.

Preferably, the trace element solution comprises at least one salt cation selected from the group consisting of Na, K, Ca, Mg, Mn, Mo, Fe, Co, Cu, Zn, and Ni, most preferably, Zn, Fe, Mn, Cu, Co, Ni, and Mo. Preferably, the trace element solution comprises at least two, at least three, at least four, at least five, at least six, at least seven, at least eight, at least nine, at least ten, or all of the salt cations selected from the group consisting of Na, K, Ca, Mg, Mn, Mo, Fe, Co, Cu, Zn, and Ni. Preferably the trace element solution comprises at least two, at least three, at least four, at least five, at least six, or all of the salt cations selected from the group consisting of Mn, Mo, Fe, Co, Cu, Zn, and Ni. Preferably, the trace element solution comprises the salt cations Zn, Fe, Mn, Cu, Co, Ni, and Mo, more preferably Zn, Mn, Cu, Co, Ni, and Mo, preferably the trace element solution comprises the salt cations Mn, Co, and Cu, preferably, Mn, Fe, and Ni, most preferably, Mn, Fe, Co, Cu, and Ni.

Preferably, the amount of each salt comprising a salt cation selected from the group consisting of Na, K, Ca, Mg, Mn, Mo, Fe, Co, Cu, Zn, and Ni in the trace element solution is in the range of 0.01% to 70%, preferably 0.1 % to 65% by weight of the chelating agent comprised in the trace element solution. Preferably, the trace element solution comprises one or more selected from Zn, Fe, Mn, Cu, Co, Ni, and Mo as salt cations and the salts comprising the salt cations are with the following amounts by weight compared to the amount of chelating agent comprised in the trace element solution: 18-17% of the salt comprising Zn, 32-43% of the salt comprising Fe, 6-14% of the salt comprising Mn, 12-27% of the salt comprising Cu, 58-68% of the salt comprising Co, 0.7-1.7% of the salt comprising Ni, and 0.08-0.25% of the salt comprising Mo. Preferably, the trace element solution comprises Zn, Fe, Mn, Cu, Co, Ni, and Mo as salt cations and the salts comprising the salt cations are with the following amounts by weight compared to the amount of chelating agent comprised in the trace element solution: 18-17% of the salt comprising Zn, 32-43% of the salt comprising Fe, 6-14% of the salt comprising Mn, 12-27% of the salt comprising Cu, 58-68% of the salt comprising Co, 0.7-1.7% of the salt comprising Ni, and 0.08-0.25% of the salt comprising Mo.

Preferably, the pH of the trace element solution is pH3-7, preferably pH3-5, preferably, pH 3-4.

Preferably, the basal medium also comprises an antifoam. Preferably, the basal medium also comprises a selection agent, e.g., an antibiotic, such as ampicillin, tetracycline, kanamycin, hygromycin, bleomycin, chloroamphenicol, streptomycin or phleomycin or a herbicide, to which the selectable marker of the cells provides resistance.

Preferably, the pH of the fermentation medium is adjusted to pH 6.6 to 9, preferably to pH 6.6 to 8.5, preferably to pH 6.6 to 7.5, more preferably to pH 7.0 to 8.5.

Preferably, the conductivity of the fermentation medium is after pH adjustment 0.1 - 100 mS/cm, preferably, 0.1-50 mS/cm, more preferably, 5 mS/cm to 50 mS/cm; even more preferably 0.5 - 25 mS/cm, further more preferably 10-25 mS/cm, most preferably 7-10 mS/cm.

In a preferred embodiment, the fermentation medium and the method using the fermentation medium is for fermentation in industrial scale. Preferably, the fermentation medium of the present invention may be useful for any fermentation having culture media of at least 20 liters, preferably, at least 50 liters, more preferably at least 300 liters, further preferred at least 1000 liters.

Another preferred embodiment is a method of cultivating a microorganism using a fermentation medium as defined herein comprising a chelating agent having the formula R¹-CH(COOX¹)-N(CH₂COOX¹)₂ (I) wherein R¹ is methyl, X¹ is (MₓH₁₋ₓ), M being a monovalent cation, preferably being selected from ammonium or alkali metal or combinations of at least two of the foregoing, x is in the range of from zero to 1, wherein x is an average value.

Disclosed herein is a method of cultivating a microorganism using a fermentation medium as defined herein comprising a chelating agent having the formula R¹-CH(COOX¹)-N(CH₂COOX¹)₂ (I) wherein R¹ is selected from hydrogen, linear or branched C₁-C₄-alkyl, phenyl, benzyl, CH₂OH, and CH₂CH₂COOX¹, X¹ is (MₓH₁₋ₓ), M being a monovalent cation, preferably being selected from ammonium or alkali metal or combinations of at least two of the foregoing, x is in the range of from zero to 1, wherein x is an average value.

Preferably, in the method of cultivating a microorganism of the present invention the chelating agent is not decomposed by the microorganism during the course of cultivation, preferably 80% to 95% of the initial amount of the chelating agent is not decomposed by the microorganism during the course of fermentation. Preferably, the amount chelating agent in the fermentation broth after fermentation compared to the fermentation medium prior the fermentation is substantially unchanged, preferably 80-100%, more preferably, 90-100%, most preferably, 95-100%.

Another preferred method of the present invention is a method of producing a compound of interest by cultivating a microorganism using a fermentation medium as defined herein comprising a chelating agent having the formula (I) R¹-CH(COOX¹)-N(CH₂COOX¹)₂ wherein R¹ is methyl, X¹ is (MₓH₁₋ₓ), M being a monovalent cation, preferably being selected from ammonium or alkali metal or combinations of at least two of the foregoing, x is in the range of from zero to 1, wherein x is an average value.

Disclosed herein is a method of producing a compound of interest by cultivating a microorganism using a fermentation medium as defined herein comprising a chelating agent having the formula (I) R¹-CH(COOX¹)-N(CH₂COOX¹)₂ wherein R¹ is selected from hydrogen, linear or branched C₁-C₄-alkyl, phenyl, benzyl, CH₂OH, and CH₂CH₂COOX¹, X¹ is (MₓH₁₋ₓ), M being a monovalent cation, preferably being selected from ammonium or alkali metal or combinations of at least two of the foregoing, x is in the range of from zero to 1, wherein x is an average value.

The fermentation may be performed as a batch, a repeated batch, a fed-batch, a repeated fed-batch or a continuous fermentation process. In a fed-batch process, either none or part of the compounds comprising one or more of the structural and/or catalytic elements, like carbon or nitrogen source, is added to the medium before the start of the fermentation and either all or the remaining part, respectively, of the compounds comprising one or more of the structural and/or catalytic elements are fed during the fermentation process. The compounds which are selected for feeding can be fed together or separate from each other to the fermentation process. In a repeated fed-batch or a continuous fermentation process, the complete start medium is additionally fed during fermentation. The start medium can be fed together with or separate from the feed(s). In a repeated fed-batch process, part of the fermentation broth comprising the biomass is removed at regular time intervals, whereas in a continuous process, the removal of part of the fermentation broth occurs continuously. The fermentation process is thereby replenished with a portion of fresh medium corresponding to the amount of withdrawn fermentation broth. In a preferred embodiment of the invention, a fed-batch fermentation process is preferred.

Many cell cultures incorporate a carbon source, like glucose, as a substrate feed in the cell culture during fermentation. Thus, preferably, the method of cultivating the microorganism comprises a feed comprising a carbon source. The carbon source containing feed can comprise a defined or a complex carbon source as described in detail herein, or a mixture thereof. Preferably, the chelating agent is comprised in the carbon source feed.

The fermentation time, pH, temperature, antifoam or other specific fermentation conditions may be applied according to standard conditions known in the art. Preferably, the fermentation conditions are adjusted to obtain maximum yields of the protein of interest.

Preferably, the temperature of the fermentation broth during fermentation is 30°C to 45°C, preferably, 35°C to 40°C. Preferably, the pH of the fermentation broth during fermentation is adjusted to pH 6.6 to 9, preferably to pH 6.6 to 8.5, preferably to pH 6.6 to 7.5, preferably 7.0 to 8.5.

Preferably, the conductivity of the fermentation broth during fermentation is 0.1 - 100 mS/cm, preferably, 0.1-50 mS/cm, more preferably, 5 mS/cm to 50 mS/cm; even more preferably 0.5 - 25 mS/cm, further more preferably 10-25 mS/cm, most preferably 7-10 mS/cm.

Preferably, fermentation is carried out with stirring and/or shaking the fermentation medium. Preferably, fermentation is carried out with stirring the fermentation medium with 50 - 2000 rpm, preferably 50 - 1600 rpm, further preferred 800 - 1400 rpm, more preferably 50 - 200 rpm.

Preferably, oxygen is added to the fermentation medium during cultivation, preferably by stirring and/or agitation as described herein or by gassing, preferably with 0-3 bar air or oxygen. Preferably, fermentation is performed under saturation with oxygen.

Preferably, the fermentation time is for 1 - 200 hours, preferably, 1 - 120 hours, more preferably 10 - 60h, even more preferably, 20 - 50h.

Preferably, the compound of interest is obtained by cultivating a microorganism, wherein preferably, the microorganism is a prokaryote or a eukaryote.

Preferably, the microorganism is a bacteria, an archaea, a fungal cell, a yeast cell or a eukaryotic cell.

Useful prokaryotes are bacterial cells such as gram positive or gram negative bacteria. Preferred useful gram positive bacteria include, but are not limited to, a Bacillus cell, e.g., Bacillus alkalophius, Bacillus amyloliquefaciens, Bacillus brevis, Bacillus circulans, Bacillus clausii, Bacillus coagulans, Bacillus firmus, Bacillus Jautus, Bacillus lentus, Bacillus licheniformis, Bacillus megaterium, Bacillus pumilus, Bacillus stearothermophilus, Bacillus subtilis, and Bacillus thurin-giensis. Preferably, the microorganism is a Bacillus cell, preferably wherein the Bacillus cell is a Bacillus cell of Bacillus subtilis, Bacillus pumilus, Bacillus licheniformis, or Bacillus lentus.

Some other preferred bacteria include strains of the order Actinomycetales, preferably, Streptomyces, preferably Streptomyces spheroides (ATTC 23965), Streptomyces thermoviolaceus (IFO 12382), Streptomyces lividans or Streptomyces murinus or Streptoverticillum verticillium ssp. verticillium. Other preferred bacteria include Rhodobacter sphaeroides, Rhodomonas palustri, Streptococcus lactis. Further preferred bacteria include strains belonging to Myxococcus, e.g., M. virescens.

Preferred gram negative bacteria are Escherichia coli and Pseudomonas sp., preferably, Pseudomonas purrocinia (ATCC 15958) or Pseudomonas fluorescens (NRRL B-11). Another preferred gram negative bacteria is Basfia succiniciproducens.

The microorganism may be a fungal cell. "Fungi" as used herein includes the phyla Ascomycota, Basidiomycota, Chytridiomycota, and Zygomycota as well as the Oomycota and Deuteromycotina and all mitosporic fungi. Representative groups of Ascomycota include, *e.g., Neurospora, Eupenicillium (=Penicillium), Emericella (=Aspergillus), Eurotium (=Aspergillus), Myceliophthora, C1,* and the true yeasts listed below. Examples of Basidiomycota include mushrooms, rusts, and smuts. Representative groups of Chytridiomycota include, *e.g., Allomyces, Blastocladiella, Coelomomyces,* and aquatic fungi. Representative groups of Oomycota include, *e.g.* Saprolegniomycetous aquatic fungi (water molds) such as *Achlya.* Examples of mitosporic fungi include *Aspergillus, e.g., Aspergillus niger, Penicillium, Candida,* and *Alternaria.* Representative groups of Zygomycota include, *e.g., Rhizopus* and *Mucor.*

Some preferred fungi include strains belonging to the subdivision Deuteromycotina, class Hyphomycetes, e.g., Fusarium, Humicola, Tricoderma, Myrothecium, Verticillum, Arthromyces, Caldariomyces, Ulocladium, Embellisia, Cladosporium or Dreschlera, in particular Fusarium oxysporum (DSM 2672), Humicola insolens, Trichoderma resii, Myrothecium verrucana (IFO 6113), Verticillum alboatrum, Verticillum dahlie, Arthromyces ramosus (FERM P-7754), Caldariomyces fumago, Ulocladium chartarum, Embellisia alli or Dreschlera halodes.

Other preferred fungi include strains belonging to the subdivision Basidiomycotina, class Basidiomycetes, e.g. Coprinus, Phanerochaete, Coriolus or Trametes, in particular Coprinus cinereus f. microsporus (IFO 8371), Coprinus macrorhizus, Phanerochaete chrysosporium (e.g. NA-12) or Trametes (previously called Polyporus), e.g. T. versicolor (e.g. PR4 28-A).

Further preferred fungi include strains belonging to the subdivision Zygomycotina, class Mycoraceae, e.g. Rhizopus or Mucor, in particular Mucor hiemalis.

The fungal host cell may be a yeast cell. "Yeast" as used herein includes ascosporogenous yeast (Endomycetales), basidiosporogenous yeast, and yeast belonging to the Fungi Imperfecti (Blastomycetes). The ascosporogenous yeasts are divided into the families Spermophthoraceae and Saccharomycetaceae. The latter is comprised of four subfamilies, Schizosaccharomycoideae *(e.g.,* genus *Schizosaccharomyces),* Nadsonioideae, Lipomycoideae, and Saccharomycoideae *(e.g.* genera *Kluyveromyces, Pichia,* and *Saccharomyces).* The basidiosporogenous yeasts include the genera *Leucosporidim, Rhodosporidium, Sporidiobolus, Filobasidium,* and *Filobasidiella.* Yeasts belonging to the Fungi Imperfecti are divided into two families, Sporobolomycetaceae *(e.g.,* genera *Sporobolomyces* and *Bullera)* and Cryptococcaceae *(e.g.* genus *Candida).* In another embodiment, the fungal host cell is a filamentous fungal cell, e.g., Ashbya spec, preferably Ashbya gossypii (Eremothecium gossypii).

The host cell may also be a eukaryote, such as a mammalian cell, an insect cell, or a plant cell.

The method of the invention can be applied for recovering any compound of interest. Thus, preferred is a method of producing a compound of interest, wherein the compound of interest is a protein.

In a preferred embodiment, the fermentation method is for production of a protein of interest at relatively high yields. Preferably, the protein of interest is expressed in an amount of at least 2 g protein (dry matter) / kg untreated fermentation medium; preferably in an amount of at least 3 g protein (dry matter) / kg untreated fermentation medium; more preferably in an amount of at least 5 g protein (dry matter) / kg untreated fermentation medium, more preferably in an amount of at least 10 g protein (dry matter) / kg untreated fermentation medium, even more preferably in an amount of at least 20 g protein (dry matter) / kg untreated fermentation medium.

Preferably, the protein of interest is an enzyme, in particular an enzyme classified as a oxidore-ductase (EC 1), a transferase (EC 2), a hydrolase (EC 3), a lyase (EC 4), a Isomerase (EC 5), or a Ligase (EC 6) (EC-numbering according to Enzyme Nomenclature, Recommendations (1992) of the Nomenclature Committee of the International Union of Biochemistry and Molecular Biology including its supplements published 1993-1999).

Most preferably, the enzyme is a hydrolase (EC 3), preferably, a glycosidase (EC 3.2) or a peptidase (EC 3.4). Especially preferred enzymes are enzymes selected from the group consisting of an amylase (in particular an alpha-amylase (EC 3.2.1.1)), a cellulase (EC 3.2.1.4), a lactase (EC 3.2.1.108), a mannanase (EC 3.2.1.25), a lipase, a phytase (EC 3.1.3.8), and a protease; in particular an enzyme selected from the group consisting of amylase, protease, lipase, mannanase, phytase, and cellulase, preferably, amylase or protease, preferably, a serine protease (EC 3.4.21). Most preferred is a serine protease. In a preferred embodiment, the protein of interest is a detergent enzyme.

In a particular preferred embodiment the following hydrolases are preferred:
Proteases: Suitable proteases include those of bacterial or fungal origin. The protease may be a serine protease or a metallo protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 and subtilisin 168 (described in WO 89/06279). Preferably, the subtilisin protease is a serine protease that uses a catalytic triad composed of Asp32, His64 and Ser221 (subtilisin BPN' numbering), preferably, the pl value of the subtilisin protease is between pH 7.0 and pH 10.0, preferably between pH 8.0 and pH 9.5. Examples of trypsin-like proteases are trypsin (e.g. of porcine or bovine origin) and the Fusarium protease described in WO 89/06270 and WO 94/25583.

Thus, preferably, the enzyme is selected from the group consisting of amylase, protease, lipase, mananase, phytase, and cellulase, preferably, amylase or protease, preferably subtilisin.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.
Additional useful proteases are described in WO2012080201 and WO2013060621.

A further preferred protease is a protease according to SEQ ID NO: 1 of DE102012215642A1 and variants thereof, wherein the preferred variants comprises one or more mutations at position 3, 4, 99, 194 and 199 (using the numbering of the alkaine protease from DSM5483), preferably comprising one or more of the following mutations: S3T, V4I, R99E, V194M, and V199I, preferably, S3T, V4I, R99E, and V199I, more preferably R99E, or R99E in combination with two additional mutations selected from the group consisting of S3T, V4I, and V199I, preferably SEQ ID NO: 1 of DE102012215642A1 with R99E, or S3T, V4I, V194M, and V199I, or S3T, V4I and V199I. A further preferred protease is a protease according to SEQ ID NO: 2 of DE102012215642A1 and variants thereof, wherein the preferred variants comprises a mutation at position 99 and an insertion between position 99 and 100, wherein the insertion is an aspartate (Asp, D) residue. In this embodiment preferably the mutation at position 99 is S99A. Further preferred protease variants are SEQ ID NO: 7 of DE102011118032A1 comprising the mutations S3T, V4I and V205I or SEQ ID NO:8 of DE102011118032A1 comprising the mutations S3T, V4I, V193M, V199I, and L211D using the numbering of the alkaine protease from DSM5483.

Preferred commercially available protease enzymes include Alcalase(TM), Savinase(TM), Primase(TM), Duralase(TM), Esperase(TM), and Kannase(TM) (Novozymes A/S), Maxatase(TM), Maxacal(TM), Maxapem(TM), Properase(TM), Purafect(TM), Purafect OxP(TM), FN2(TM), and FN3(TM) (Genencor International Inc.).
Lipases: Suitable lipases include those of bacterial or fungal origin. Examples of useful lipases include lipases from Humicola (synonym Thermomyces), e.g. from H. lanuginosa (T. lanuginosus), as described in EP 258 068 and EP 305 216 or from H. insolens as described in WO 96/13580, a Pseudomonas lipase, e.g. from P. alcaligenes or P. pseudoalcaligenes (EP 218 272), P. cepacia (EP 331 376), P. stutzeri (GB 1,372,034), P. fluorescens, Pseudomonas sp. strain SD 705 (WO 95/06720 and WO 96/27002), P. wisconsinensis (WO 96/12012), a Bacillus lipase, e.g. from B. subtilis (Dartois et al. (1993), Biochemica et Biophysica Acta, 1131, 253-360), B. stearothermophilus (JP 64/744992) or B. pumilus (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipolase(TM) and Lipolase Ultra(TM) (Novozymes A/S).

Amylases: Suitable amylases (alpha and/or beta) include those of bacterial or fungal origin. Chemically modified or protein engineered mutants are included. Amylases include, for example, alpha-amylases obtained from Bacillus, e.g. a special strain of B. licheniformis, described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl(TM), Termamyl(TM), Fungamyl(TM) and BANT(TM) (Novozymes A/S), Rapidase(TM) and Purastar(TM) (from Genencor International Inc.).

Cellulases: Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g. the fungal cellulases produced from Humicola insolens, Myceliophthora thermophila and Fusarium oxysporum disclosed in U.S. Pat. Nos. 4,435,307, U.S. 5,648,263, U.S. 5,691,178, U.S. 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, U.S. Pat. Nos. 5,457,046, U.S. 5,686,593, U.S. 5,763,254, WO 95/24471, WO 98/12307 and PCT/DK98/00299.

Commercially available cellulases include Celluzyme(TM), and Carezyme(TM) (Novozymes A/S), Clazinase(TM), and Puradax HA(TM) (Genencor International Inc.), and KAC-500 (B)(TM) (Kao Corporation).

Oxidoreductases: Oxidoreductases that may be treated according to the invention include peroxidases, and oxidases such as laccases.

Peroxidases: An enzyme exhibiting peroxidase activity may be any peroxidase enzyme comprised by the enzyme classification (EC 1.11.1.7), or any fragment derived therefrom, exhibiting peroxidase activity.

Particularly, a recombinantly produced peroxidase is preferred, e.g., a peroxidase derived from a Coprinus sp., in particular C. macrorhizus or C. cinereus according to WO 92/16634, or a variant thereof, e.g., a variant as described in WO 93/24618 and WO 95/10602.

Laccases and Laccase related enzymes: In the context of this invention, laccases and laccase related enzymes contemplate any laccase enzyme comprised by the enzyme classification (EC 1.10.3.2), any chatechol oxidase enzyme comprised by the enzyme classification (EC 1.10.3.1), any bilirubin oxidase enzyme comprised by the enzyme classification (EC 1.3.3.5) or any monophenol monooxygenase enzyme comprised by the enzyme classification (EC 1.14.18.1). The microbial laccase enzyme may be derived from bacteria or fungi (including filamentous fungi and yeasts) and suitable examples include a laccase derivable from a strain of Aspergillus, Neurospora, e.g., N. crassa, Podospora, Botrytis, Collybia, Fomes, Lentinus, Pleurotus, Trametes, e.g., T. villosa and T. versicolor, Rhizoctonia, e.g., R. solani, Coprinus, e.g. C. plicatilis and C. cinereus, Psatyrella, Myceliophthora, e.g. M. thermophila, Schytalidium, Polyporus, e.g., P. pinsitus, Phlebia, e.g., P. radita (WO 92/01046), or Coriolus, e.g., C. hirsutus (JP 2-238885), in particular laccases obtainable from Trametes, Myceliophthora, Schytalidium or Polyporus.

Further, protein engineered variants of a protein of interest, made by recombinant DNA techniques or by chemical modification, may be of particular interest.

Another embodiment of the present invention is the use of a chelating agent having the formula (I)

R¹-CH(COOX¹)-N(CH₂COOX¹)₂

wherein
R¹ is methyl,
X1 is (MxH1-x), M being a monovalent cation, preferably being selected from ammonium or alkali metal or combinations of at least two of the foregoing,
x is in the range of from zero to 1, wherein x is an average value, as described in detail herein,
for the preparation of a fermentation medium, preferably as described herein in more details.

Disclosed herein is the use of a chelating agent having the formula (I)

R¹-CH(COOX¹)-N(CH₂COOX¹)₂

wherein
R¹ is selected from hydrogen, linear or branched C₁-C₄-alkyl, phenyl, benzyl, CH₂OH, and CH₂CH₂COOX¹,
X1 is (MxH1-x), M being a monovalent cation, preferably being selected from ammonium or alkali metal or combinations of at least two of the foregoing,
x is in the range of from zero to 1, wherein x is an average value, as described in detail herein,
for the preparation of a trace element solution or a fermentation medium, preferably as described herein in more details.

Another embodiment of the present invention is the use of a chelating agent having the formula (I) R¹-CH(COOX¹)-N(CH₂COOX¹)₂ wherein R¹ is methyl, X¹ is (MₓH₁₋ₓ), M being a monovalent cation, preferably being selected from ammonium or alkali metal or combinations of at least two of the foregoing, x is in the range of from zero to 1, wherein x is an average value, as described in detail herein, for the fermentative production of a compound of interest, preferably a protein, in particular preferred an enzyme, as described herein.

Also disclosed herein is the use of a chelating agent having the formula (I) R¹-CH(COOX¹)-N(CH₂COOX¹)₂ wherein R¹ is selected from hydrogen, linear or branched C₁-C₄-alkyl, phenyl, benzyl, CH₂OH, and CH₂CH₂COOX¹, X¹ is (MₓH₁₋ₓ), M being a monovalent cation, preferably being selected from ammonium or alkali metal or combinations of at least two of the foregoing, x is in the range of from zero to 1, wherein x is an average value, as described in detail herein, for the fermentative production of a compound of interest, preferably a protein, in particular preferred an enzyme, as described herein.

Another embodiment of the present invention is the use of a chelating agent having the formula (I)

R¹-CH(COOX¹)-N(CH₂COOX¹)₂

wherein
R¹ is methyl,
X1 is (MxH1-x), M being a monovalent cation, preferably being selected from ammonium or alkali metal or combinations of at least two of the foregoing,
x is in the range of from zero to 1, wherein x is an average value, as described in detail herein,
for the preparation of a fermentation medium, preferably as described herein in more details.

Disclosed herein is the use of a chelating agent having the formula (I)

R¹-CH(COOX¹)-N(CH₂COOX¹)₂

wherein
R¹ is selected from linear or branched C₁-C₄-alkyl, phenyl, benzyl, and CH₂OH,
X1 is (MxH1-x), M being a monovalent cation, preferably being selected from ammonium or alkali metal or combinations of at least two of the foregoing,
x is in the range of from zero to 1, wherein x is an average value, as described in detail herein,
for the preparation of a trace element solution or a fermentation medium, preferably as described herein in more details.

Disclosed herein is the use of a chelating agent having the formula (I)

R¹-CH(COOX¹)-N(CH₂COOX¹)₂

wherein
R¹ is selected from linear or branched C₁-C₄-alkyl, preferably methyl,
X1 is (MxH1-x), M being a monovalent cation, preferably being selected from ammonium or alkali metal or combinations of at least two of the foregoing,
x is in the range of from zero to 1, wherein x is an average value, as described in detail herein,
for the preparation of a trace element solution or a fermentation medium, preferably as described herein in more details.

The compound of interest resulting from the method of the present invention may be purified from the fermentation broth and processed by methods known in the art.

The invention is further illustrated in the following examples which are not intended to be in any way limiting to the scope of the invention as claimed.

### Examples

The following examples only serve to illustrate the invention. The numerous possible variations that are obvious to a person skilled in the art also fall within the scope of the invention.

Unless otherwise stated the following experiments have been performed by applying standard equipment, methods, chemicals, and biochemicals as used in genetic engineering and fermentative production of chemical compounds by cultivation of microorganisms. See also Sambrook et al. (Molecular Cloning: A Laboratory Manual. 2nd edition, Cold Spring Harbor Laboratory, Cold Spring Harbor Laboratory Press, Cold Spring Harbor, NY, 1989) and Chmiel et al. (Bioprocesstechnik 1. Einführung in die Bioverfahrenstechnik, Gustav Fischer Verlag, Stuttgart, 1991).

### Examples

### Example 1:

Comparison of trace element solutions with different chelating agents to state of the art chelating agent citric acid regarding precipitation and color formation under different temperature and pH conditions.

### Preparation trace element solutions:

A trace element solution was mixed together with one of the following chelating agents: citric acid, NTA, MGDA, GLDA, EDTA, DTPA, EGTA, or HEEDTA. Therefore, at first each chelating agent was weight equimolar to the utilized divalent cations.

The pH was measured and was adjusted with 25% NaOH or 20% H2SO4 to pH 7.0. Subsequently, the salts (NH4)2Fe(SO4)2*6H2O, CuSO4*5H20, ZnSO4*7H2O, MnSO4*H2O, Co(NO3)2, NiSO4*6H2O and Na2MoO4*2H2O were added. After each compound supplementation the solutions were characterized regarding pH value, color and precipitation. After the addition of the last compound, i.e., Na2MoO4, the solutions were aliquoted. Each one sample was stored for 24 h at room temperature and one sample at 4°C. Afterwards samples were again characterized by pH value, color and precipitation behavior. The pH values were readjusted to pH 6 and the precipitation investigated.

### Results:

Trace element solution with citric acid as chelating agent showed after addition of Na2MoO4 and the final mixing a color development to an emerald green and was a crystal clear solution.

The measured pH value was pH 4.6. After storage for 24 h precipitates formed a red pellet. This status got even more pronounced after pH adjustment to pH 6.

Trace element solution with nitrilotriacetic acid (NTA) as chelating agent showed after addition of Na2MoO4 and the final mixing a color development to an emerald green and was a crystal clear solution. The measured pH value was pH 2.5. Neither after storage time of 24 h nor after pH adjustment to pH of 6 any precipitates occurred.

Trace element solution with methylglycinediacetic acid (MGDA) as chelating agent showed after addition of Na2MoO4 and the final mixing a color development to green blue and crystal clear solution. The measured pH value was pH 2.8. Neither after storage for 24 h nor after pH adjustment to pH of 6 any precipitates occurred.

Trace element solution with glutamic acid N,N-diacetic acid (GLDA) as chelating agent showed after addition of Na2MoO4 and the final mixing a color development to light blue and was a crystal clear solution. The measured pH value was pH 3.7. After storage for 3 h at 4°C the solution turned to green color and precipitates were detected. After 24 h at room temperature and pH readjustment to pH 6 solution color turned to brown and precipitates were formed.

Trace element solution with ethylenediaminetetraacetic acid (EDTA) as chelating agent showed after addition of CuSO4 a color development from light yellow to light blue. With the supplementation of CuSO4 strong white precipitates occurred. After pH adjustment to pH 4 the precipitates were dissolved and the color development after final mixing turned to a blue and clear solution. The measured pH value was pH 3. After storage for 24 h and pH adjustment to pH 6 no further precipitation was noticed.

Trace element solution with diethylene triamine pentaacetic acid (DTPA) as chelating agent showed after addition of Na2MoO4 and the final mixing a color development to a dark green and was a clear solution. The measured pH value was pH 2.6. After storage for 24 h and pH adjustment to pH 6 no precipitation was detected.

Trace element solution with ethylene glycol tetraacetic acid (EGTA) as chelating agent showed after addition of Na2MoO4 and the final mixing a color development to dark blue and was a clear solution. The measured pH value was pH 2.7. After storage for 24 h and after pH adjustment to pH 6 the solution turned darker and brownish.

Trace element solution with hydroxyethylethylenediamine triacetic acid (HEEDTA) as chelating agent showed after addition of Na2MoO4 and the final mixing a color development to a dark blue and was a clear solution. The measured pH value was pH 2.6. After storage for 24 h and pH adjustment to pH 6 no precipitation was detected.

Summarizing in terms of complexation and precipitation the chelating agents NTA, MGDA, DTPA and HEEDTA showed better results than citrate.

### Example 2:

Comparison of fermentation medium with trace element solutions with different chelating agents to state of the art chelating agent citric acid regarding precipitation and color formation under different temperature and pH conditions.

### Preparation of medium with trace element solutions with different chelating agents:

A fermentation medium was mixed with different chelating agents. Therefore, deionized water, Ca(NO3)2 * 4 H2O, MgSO4 *7H2O, NH4H2PO4, and KH2PO4 were mixed together in this certain order with the respective chelating agent. Each chelating agent was weight equimolar to the utilized divalent cations. Afterwards the pH is measured. All three media were aliquoted six times each. Three aliquots of each medium were adjusted to pH 6.7, the other three samples to pH 7.7. Samples were mixed with the trace element solutions as described in Example 1 and stored at 35°C, room temperature and 4°C for 24 h. Then color formation and precipitation were characterized.

### Results:

The fermentation medium with citric acid as chelating agent showed strong precipitations in all samples under different temperature and pH conditions.
The fermentation medium with NTA and MGDA as chelating agent showed low precipitations for samples with pH adjustment to 6. For samples with a pH adjustment to 7.7 no precipitations occurred. Under the different temperature conditions no difference in precipitation was detected.

### Example 3:

Comparison of medium with trace element solutions with different chelating agents regarding biomass growth and product formation in a small-scale cultivation.

### Microtiter plate cultivations:

For the comparable cultivations online monitored microtiter plate cultivation system (Biolector, m2p labs) was used. Glucose was supplied in total in an amount of 14 g/l for a cultivation time of 48 h. The trace element solution was composed as described above and the chelating agents citrate, MGDA, NTA, EDTA, DTPA, HEEDTA, or EGTA were supplemented equimolar in each approach in replicates. pH value of the trace element solution was adjusted to pH 6.7 and pH 7.7 respectively for two approaches. As cultivation medium 1 g/L trace element solution with one of the chelating agents, 24 g/L KH2PO4, 2 mL/L MgSO4*7H2O, 8 mL/L 8% Ca(NO3)2 solution and 5.6 g/L (NH4)2NO3 were supplemented. The pH was adjusted to pH 7.3 with sodium hydroxide and sterilized for 30 min at 121°C, 1 bar over pressure. The different fermentation media were aliquoted to a BioLector Flowerplate/microtiter plate (m2p labs) 700 µL each and cultivated at 30°C, shaker frequency 1200 rpm for 48 h after inoculating with Bacillus licheniformis producing a subtilisin protease. During the cultivation oxygen partial pressure, pH and scattered light for biomass growth are measured online by optical sensors every 15 min.

### Product formation:

For characterization of protease formation enzyme activity was measured by casein based activity assay.

### Results:

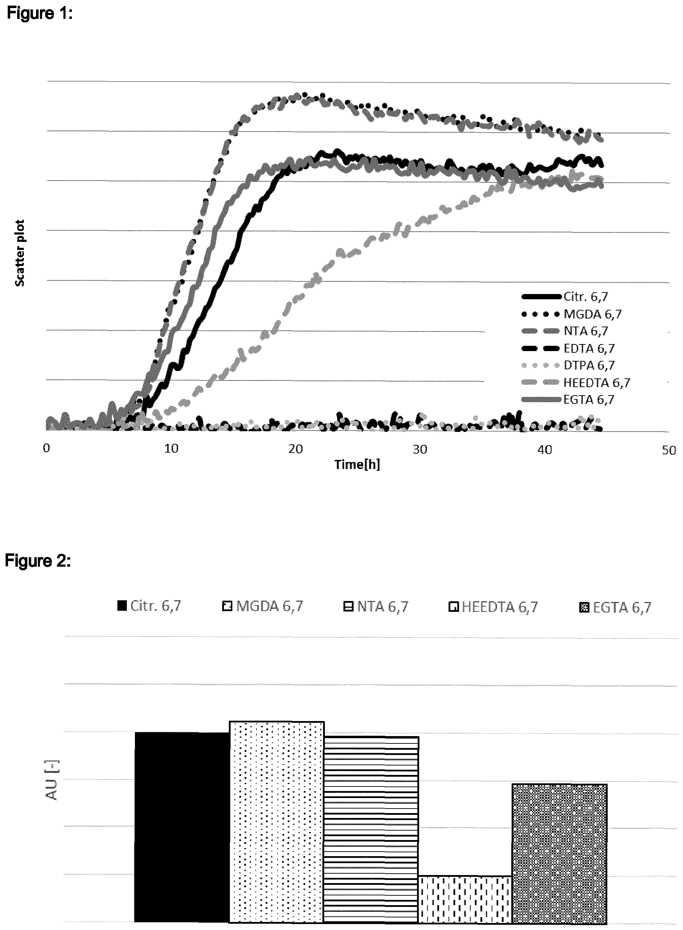

Development of biomass was measured as scatter plot, which showed fastest growth and highest values with media wherein MGDA or NTA were supplemented as chelating agents. Compared to these for cultivations wherein citrate or EGTA were used as chelating agents the biomass growth started delayed and maximal values were about 20% lower. HEEDTA as chelating agent showed strong delayed growth and lower biomass development and within cultivations with EDTA or DTPA as chelating agents no growth was detected.
Cultivations with supplementation of MGDA or NTA as chelating agents in the trace element solution and medium showed better (20% higher scatter) development for growth and maximal scatter values compared to citric acid, EGTA, HEEDTA and especially DTPA and EDTA (cf. Figure 1).

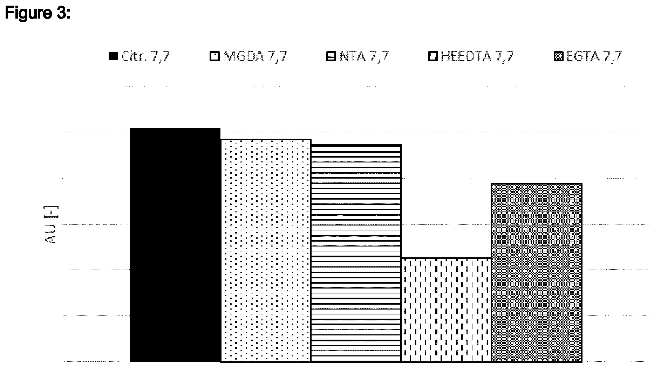

Cultivations with variated chelating agents resulted in different protease activities and consequently different enzyme formation was measured (cf. Figures 2 and 3, normalized activity units, AU). Enzyme activities for MGDA and NTA were on a comparable level as the prior art standard citrate. The result for EGTA as chelating agent was about 25% lower and for HEEDTA as chelating agent even 60% lower. The results were comparable for both pH values.

### Example 4:

Comparison of medium with trace element solutions with different chelating agents regarding biomass growth and product formation in a large-scale cultivation.

### Cultivation process in 20 L scale:

The fermentation processes were conducted in a baffled stirred tank reactor (STR) with pH, pO2 and temperature probes in a medium with glucose as main carbon source and complex plant protein as N-source. The concentration of O2 and CO2 were monitored by gas analyses. For the cultivations, the fedbatch mode was chosen with a start working volume of 10 L. As bioreactors, 21 L steal vessels were used with 3 rushton turbines with 6 blades.
Medium preparation and sterilization for seed and main culture medium took place in the bioreactor, respectively. The medium contained complex plant protein, NH4H2PO4, KH2PO4, Mn(II)SO4 *H2O, Fe(II)SO4* 7H2O, MgSO4*7H2O, Ca(NO3)2* 4 H2O, trace element solution as described above, antifoam and 2 mL/L MGDA or citric acid. pH was adjusted to pH 6.5. The medium was sterilized in the bioreactor under stirred conditions (600 rpm) for 60 min at 121°C. For inoculation with Bacillus licheniformis producing a subtilisin protease 2% of the starting working volume was used from a seed culture fermenter with the same medium. Seed culture cultivation was conducted at 37°C and harvested during exponential growth phase after 16 h. The main cultivation was conducted at 37°C, with pO2 dependent stirred cascade from 350 - 1400 rpm, pH 7.7 and an aeration rate of 1.5 vvm. The glucose feed was started 8 h after inoculation.

**Table 1: Characteristic fermentation indicators (normalized to %) for biomass growth (CTR [mmol/L *h]) and protease formation (Titer [g/L], space time yield (STY) [g/L*h], Titer/CTR) for MGDA as chelating agent compared to citric acid as chelating agent.**

| | **w/ Citric acid** | **w/ MGDA** |
|---|---|---|
| Titer (productivity) | 100% | 104% |
| STY (productivity) | 100% | 110% |

As shown above, higher product titer and space time yields (STY) were measured in the fermentation processes with utilization of MGDA as chelating agent.

The concentration of MGDA in the fermentation broth after cultivation of B. licheniformis was determined and was compared after calculating the respective dilutions steps with the concentration of the MGDA in the fermentation medium before the cultivation. The concentration of MGDA was measured potentiometrically by titration using a FeCI solution. The amount of MGDA degraded during the course of fermentation was below 5%.

## Claims

1. A fermentation medium comprising a basal medium and a chelating agent having the formula (I)
R¹-CH(COOX¹)-N(CH₂COOX¹)₂
wherein
R¹ is methyl,
X¹ is (MₓH₁₋ₓ), M being a monovalent cation, preferably being selected from ammonium or alkali metal or combinations of at least two of the foregoing and x being in the range of from zero to 1, wherein x is an average value.

2. The fermentation medium of any of the proceeding claims, wherein M of formula (I) is sodium or potassium or a combination thereof.

3. The fermentation medium of any of the proceeding claims, wherein x of formula (I) is 1.

4. The fermentation medium of any of the proceeding claims, wherein the fermentation medium does not comprise one or more of the compounds selected from the group consisting of citric acid, EDTA, DTPA, HEEDTA, and EGTA and/or a salt thereof.

5. The fermentation medium of any of the proceeding claims, wherein the basal medium comprises one or more components selected from the group consisting of nitrogen source, phosphor source, sulfur source and salt, and optionally one or more further components selected the group consisting of micronutrients, like vitamins, trace elements, and antifoam.

6. The fermentation medium of any of the proceeding claims, wherein the fermentation medium comprises a carbon source.

7. The fermentation medium of any of the proceeding claims, wherein the fermentation medium is for fermentation in industrial scale.

8. A method of cultivating a microorganism using a fermentation medium as defined in any of claims 1-7.

9. A method of producing a compound of interest by cultivating a microorganism using a fermentation medium as defined in any of claims 1-7.

10. The method of claim 8 or 9, wherein the microorganism is a Bacillus cell, preferably wherein the Bacillus cell is a Bacillus cell of Bacillus subtilis, Bacillus pumilus, Bacillus licheniformis, or Bacillus lentus.

11. The method of any of claims 8-10, wherein the compound of interest is a protein, wherein preferably the protein of interest is an enzyme, preferably selected from the group consisting of amylase, protease, lipase, mananase, phytase, and cellulase, preferably, amylase or protease, most preferably subtilisin.

12. Use of a chelating agent having the formula (I)
R¹-CH(COOX¹)-N(CH₂COOX¹)₂
wherein
R¹ is methyl,
X¹ is (MₓH₁₋ₓ), M being a monovalent cation, preferably being selected from ammonium or alkali metal or combinations of at least two of the foregoing and x being in the range of from zero to 1, wherein x is an average value,
for the preparation of a fermentation medium.

## Patentansprüche

1. Fermentationsmedium, umfassend ein Grundmedium und einen Chelatbildner mit der Formel (I)
R¹-CH(COOX¹)-N(CH₂COOX¹)₂
wobei
R¹ für Methyl steht,
X¹ für (MₓH₁₋ₓ) steht, wobei M für ein einwertiges Kation steht, das vorzugsweise aus Ammonium oder Alkalimetall oder Kombinationen von mindestens zwei davon ausgewählt ist, und x im Bereich von null bis 1 liegt, wobei x ein Durchschnittswert ist.

2. Fermentationsmedium nach einem der vorhergehenden Ansprüche, wobei M von Formel (I) für Natrium oder Kalium oder eine Kombination davon steht.

3. Fermentationsmedium nach einem der vorhergehenden Ansprüche, wobei x der Formel (I) für 1 steht.

4. Fermentationsmedium nach einem der vorhergehenden Ansprüche, wobei das Fermentationsmedium nicht eine oder mehrere der Verbindungen aus der Gruppe bestehend aus Citronensäure, EDTA, DTPA, HEEDTA und EGTA und/oder ein Salz davon umfasst.

5. Fermentationsmedium nach einem der vorhergehenden Ansprüche, wobei das Grundmedium eine oder mehrere Komponenten aus der Gruppe bestehend aus Stickstoffquelle, Phosphorquelle, Schwefelquelle und Salz und gegebenenfalls eine oder mehrere weitere Komponenten aus der Gruppe bestehend aus Mikronährstoffen, wie Vitaminen, Spurenelementen und Antischaummitteln umfasst.

6. Fermentationsmedium nach einem der vorhergehenden Ansprüche, wobei das Fermentationsmedium eine Kohlenstoffquelle umfasst.

7. Fermentationsmedium nach einem der vorhergehenden Ansprüche, wobei das Fermentationsmedium zur Fermentation in technischem Maßstab dient.

8. Verfahren zum Kultivieren eines Mikroorganismus unter Verwendung eines Fermentationsmediums gemäß einem der Ansprüche 1-7.

9. Verfahren zur Herstellung einer Verbindung von Interesse durch Kultivieren eines Mikroorganismus unter Verwendung eines Fermentationsmediums gemäß einem der Ansprüche 1-7.

10. Verfahren nach Anspruch 8 oder 9, wobei es sich bei dem Mikroorganismus um eine Bacilluszelle handelt, vorzugsweise wobei es sich bei der Bacilluszelle um eine Bacilluszelle von Baccilus subtilis, Bacillus pumilus, Bacillus licheniformis oder Bacillus lentus handelt.

11. Verfahren nach einem der Ansprüche 8-10, wobei es sich bei der Verbindung von Interesse um ein Protein handelt, wobei es sich vorzugsweise bei dem Protein von Interesse um ein Enzym handelt, vorzugsweise ausgewählt aus der Gruppe bestehend aus Amylase, Protease, Lipase, Mananase, Phytase und Cellulase, vorzugsweise Amylase oder Protease, ganz besonders bevorzugt Subtilisin.

12. Verwendung eines Chelatbildners mit der Formel (I)
R¹-CH(COOX¹)-N(CH₂COOX¹)₂
wobei
R¹ für Methyl steht,
X¹ für (MₓH₁₋ₓ) steht, wobei M für ein einwertiges Kation steht, das vorzugsweise aus Ammonium oder Alkalimetall oder Kombinationen von mindestens zwei davon ausgewählt ist, und x im Bereich von null bis 1 liegt, wobei x ein Durchschnittswert ist,
zur Herstellung eines Fermentationsmediums.

## Revendications

1. Milieu de fermentation comprenant un milieu de base et un agent chélatant répondant à la formule (I)
R¹-CH(COOX¹)-N(CH₂COOX¹)₂
dans lequel
R¹ est un groupe méthyle,
X¹ est (MₓH₁₋ₓ), M étant un cation monovalent, de préférence qui est choisi entre l'ion ammonium et un métal alcalin ou des combinaisons d'au moins deux des cations précédents, et x étant dans la plage de zéro à 1, x étant une valeur moyenne.

2. Milieu de fermentation selon l'une quelconque des revendications précédentes, dans lequel M de la formule (I) est le sodium ou le potassium ou une combinaison de ceux-ci.

3. Milieu de fermentation selon l'une quelconque des revendications précédentes, dans lequel x de la formule (I) vaut 1.

4. Milieu de fermentation selon l'une quelconque des revendications précédentes, le milieu de fermentation ne comprenant pas un ou plusieurs des composés choisis dans le groupe constitué par l'acide citrique, l'EDTA, le DTPA, l'HEEDTA et l'EGTA et/ou un sel de ceux-ci.

5. Milieu de fermentation selon l'une quelconque des revendications précédentes, dans lequel le milieu de base comprend un ou plusieurs composants choisis dans le groupe constitué par une source d'azote, une source de phosphore, une source de soufre et un sel et éventuellement un ou plusieurs autres composants choisis dans le groupe constitué par les micronutriments, comme des vitamines, des oligo-éléments et un antimousse.

6. Milieu de fermentation selon l'une quelconque des revendications précédentes, le milieu de fermentation comprenant une source de carbone.

7. Milieu de fermentation selon l'une quelconque des revendications précédentes, le milieu de fermentation étant destiné à une fermentation à l'échelle industrielle.

8. Procédé de culture d'un micro-organisme à l'aide d'un milieu de fermentation tel que défini dans l'une quelconque des revendications 1-7.

9. Procédé de production d'un composé présentant de l'intérêt par la culture d'un micro-organisme à l'aide d'un milieu de fermentation tel que défini dans l'une quelconque des revendications 1-7.

10. Procédé selon la revendication 8 ou 9, dans lequel le micro-organisme est une cellule de *Bacillus,* de préférence dans lequel la cellule de *Bacillus* est une cellule de *Bacillus* de *Bacillus subtilis,* de *Bacillus pumilus,* de *Bacillus licheniformis* ou de *Bacillus lentus.*

11. Procédé selon l'une quelconque des revendications 8-10, dans lequel le composé présentant de l'intérêt est une protéine, dans lequel de préférence la protéine présentant de l'intérêt est une enzyme, de préférence choisie dans le groupe constitué par une amylase, une protéase, une lipase, une mananase, une phytase et une cellulase, de préférence une amylase ou une protéase, le plus préférablement la subtilisine.

12. Utilisation d'un agent chélatant répondant à la formule (I)
R¹-CH(COOX¹)-N(CH₂COOX¹)₂
dans laquelle
R¹ est un groupe méthyle,
X¹ est (MₓH₁₋ₓ), M étant un cation monovalent, de préférence qui est choisi entre l'ion ammonium et un métal alcalin ou des combinaisons d'au moins deux des cations précédents, et x étant dans la plage de zéro à 1, x étant une valeur moyenne,
pour la préparation d'un milieu de fermentation.
